# EUROPEAN PATENT APPLICATION

(11) **EP 1 731 954 A1**
(43) Date of publication of application: **13.12.2006**
(21) Application number: 05721427.2
(22) Date of filing: 17.03.2005
(51) Int. Cl.: G03B 15/00, G02B 7/10, G03B 17/12, G06F 15/00, H04N 5/232, A61B 5/117

(54) **EYE IMAGE CAPTURING DEVICE AND PORTABLE TERMINAL**

(30) Priority: 01.04.2004 JP 2004108853
(71) Applicant: Matsushita Electric Industries Co., Ltd., Kadoma-shi, Osaka 571-8501 (JP)
(72) Inventor: WAKAMORI, Masahiro, Kanagawa 226-0013 (JP)
(74) Representative: Grünecker, Kinkeldey, Stockmair & Schwanhäusser Anwaltssozietät
(86) International application number: PCT/JP2005/005450
(87) International publication number: WO 2005/098531

(57) **Abstract**

The eye-imaging device includes: an imager (200) capable of switching over between a telephoto mode to take eye-images and a wide-angle mode to take facial images, keeping a certain distance between the eye and camera; a display (390) to display images taken by the wide-angle mode; a guide checker (330) to check automatically that the eye has been guided to a predetermined position according to the image taken by the wide-angle mode; and a lens selector (230) to switch over the imager (200) automatically from the wide-angle mode to the telephoto mode when the guide checker (330) checks that the eye has been guided to the predetermined position.

## Description

### TECHNICAL FIELD

The present invention relates to an eye-imaging device installed in an iris authenticator or the like and particularly to an eye-imaging device capable of taking both facial images with wide angle-of-view and iris images with narrow angle-of-view, and a portable terminal device using the same.

### BACKGROUND ART

In recent years, cell-phones installed with a wide-angle camera have become popular that can function to take landscape photos to send them to correspondents or can be used as a videophone.

Meanwhile, along with the popularity of payment system using portable terminal devices such as cell-phones or the like, an iris authenticator capable of performing highly reliable personal authentication has been tried installed into the portable terminal devices. The iris authentication is generally performed through: illuminating an eye and around the eye of a subject person with near infrared light; taking images of the eye and around the eye (hereafter referred to as eye-images); and extracting iris data from the eye-images taken to collate them with the iris data that have been already registered in the iris data-base for the personal authentication. The eye-imaging device used here should be a telephoto camera with a narrow angle-of-view to extract accurate iris data of the subject person. Most lenses are usually provided with a visible light protection filter as the sharpest iris image can be taken in near infrared light region. An imaging device occupying only a small space to install it in a portable terminal device is disclosed that functions as a wide-angle camera capable of taking landscape or facial photos as well as a narrow angle-of-view telephoto camera capable of taking eye-images (see for instance Japanese Patent Unexamined Publication No. 2003-256819).

However, the telephoto camera can hardly be used for guiding an eye in a display screen due to its narrow angle-of-view and a large mirror to guide the eye is difficult to dispose due to restrictions of setting area. In conventional way, the eye must usually be near to the camera to a distance of 5 to 15 cm for taking eye-images. In this way, the problems are that the subject person's eye must near to the camera so closely to take images for iris authentication that he/she feels wrongness or uneasiness in handling the camera significantly, and that others may easily sense his/her behaviors for the iris authentication.

### SUMMARY OF THE INVENTION

To solve the aforementioned problems, the present invention aims at providing an eye-imaging device and a portable terminal device using the same that can perform the iris authentication, keeping a certain distance between a subject person's eye and a camera, which can eliminate the feeling of wrongness or uneasiness in handling the camera and can prevent others from sensing his/her behaviors for the iris authentication.

The eye-imaging device in the present invention comprises: an imager capable of switching over between a telephoto mode to take eye-images and a wide-angle mode to take facial images of a subject person, keeping a certain distance between the subject person's eye and the camera; a display to display images taken by the wide-angle mode; a guide checker to check automatically that the subject person's eye has been guided to a predetermined position according to the image taken by the wide-angle mode; and a selector to switch over the imager automatically from the wide-angle mode to the telephoto mode when the guide checker checks that the subject person's eye has been guided properly to the predetermined position.

Because the subject person's eye is not required to near the camera too closely for iris authentication, the configuration can eliminate the feeling of wrongness or uneasiness in handling the camera to take eye-images and can prevent others from sensing his/her behaviors for iris authentication.

It is another phase of the present invention that an imager of the eye-imaging device can use a telephoto lens and a wide-angle lens. The imager is provided with a lens selector to switch over between the telephoto mode and the wide-angle mode by switching over the telephoto lens and the wide-angle lens. The configuration can realize a inexpensive selector because the switchover between the telephoto mode and the wide-angle mode is done by the lens only.

It is still another phase of the present invention that an imager of the eye-imaging device can use a zoom lens. The imager is provided with a zoom lens driver to switch over between the telephoto mode and the wide-angle mode by driving the zoom lens. The user can adjust the eye position easily as soon as it begins to deflect because he/she can always watch the eye position in the zooming images continuously in display.

It is still another phase of the present invention that an imager of the eye-imaging device can use a telephoto camera and a wide-angle camera. The imager is provided with a selector to switch over between the telephoto mode and the wide-angle mode by switching over the telephoto camera and the wide-angle camera. The configuration can realize a highly reliable and power saving eye-imaging device because the imager has not any driving mechanism.

The portable terminal device disclosed in the present invention is installed with the aforementioned eye-imaging device.

### BRIEF DESCRIPTION OF THE DRAWINGS

FIG. 1 shows schematically a foldable cell-phone at unfolded state installed with the eye-imaging device and iris authentication processor used in the preferred embodiment of the present invention.
FIG. 2 shows a cross-sectional view taken along the line 1 - 1 of the foldable cell-phone.
FIG. 3 shows a block diagram of the eye-imaging device and iris authentication processor.
FIG. 4 is a flowchart showing an operation of the eye-imaging device.
FIG. 5 shows an angle-of-view of the telephoto lens of the imager in the eye-imaging device.
FIG. 6 shows an angle-of-view of the wide-angle lens of the imager in the eye-imaging device.
FIG. 7 shows a block diagram of the eye-imaging device and iris authentication processor used in the second preferred embodiment of the present invention.
FIG.8 is a flowchart showing an operation of the eye-imaging device.
FIG.9 shows a block diagram of the eye-imaging device and iris authentication processor used in the third preferred embodiment of the present invention.

### DETAILED DESCRIPTION OF THE PREFERRED EMBODIMENTS

Now, eye-imaging devices used in the preferred embodiments of the present invention are described with reference to drawings.

### (The first preferred embodiment)

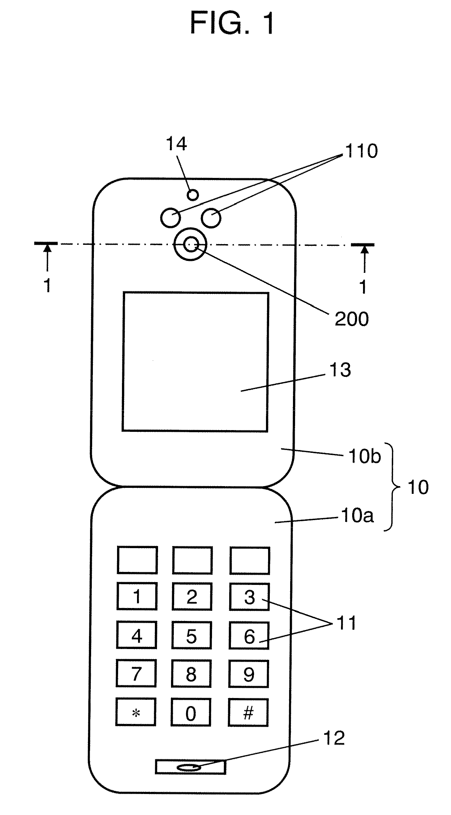

FIG. 1 shows a schematic view of foldable cell-phone 10 at an unfolded state installed with the eye-imaging device and iris authentication processor used in the preferred embodiment of the present invention. Cell-phone 10 comprises first body 10a and second body 10b connected foldably freely. First body 10a is provided with keys 11, microphone 12 and others. Second body 10b is provided with display 13 composed of LCD, organic EL or the like. Second body 10b is provided with speaker 14, two pieces of LED 110 emitting near infrared light and imager 200 additionally.

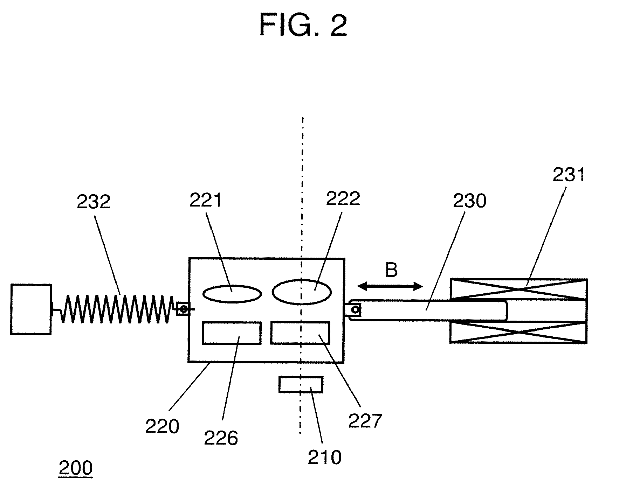

FIG. 2 is a cross-sectional view taken along the line 1 - 1 of foldable cell-phone 10 to show imager 200 schematically. In the first preferred embodiment, imager 200 comprises imaging element 210 composed of CCD, CMOS or the like and optical system 220 positioned in front of imaging element 210. Lens selector 230 including an actuator composed of solenoid 231 and coiled spring 232 slides optical system 220 in the direction indicated by arrow B. In optical system 220, telephoto lens 221 with a narrow angle-of-view and wide-angle lens 222 with a wide angle-of-view are disposed in parallel with each other, and lens selector 230 selects them so that one of telephoto lens 221 and wide-angle lens 222 is positioned in front of imaging element 210. Optical system 220 can thus switch over between telephoto mode and wide-angle mode. Visible light protection filter 226 is disposed on top of telephoto lens 221 and infrared protection filter 227 is disposed on top of wide-angle lens 222 in optical system 220. These filters are to take into imaging element 210 only infrared light to telephoto lens 221 and only visible light to wide-angle lens 222.

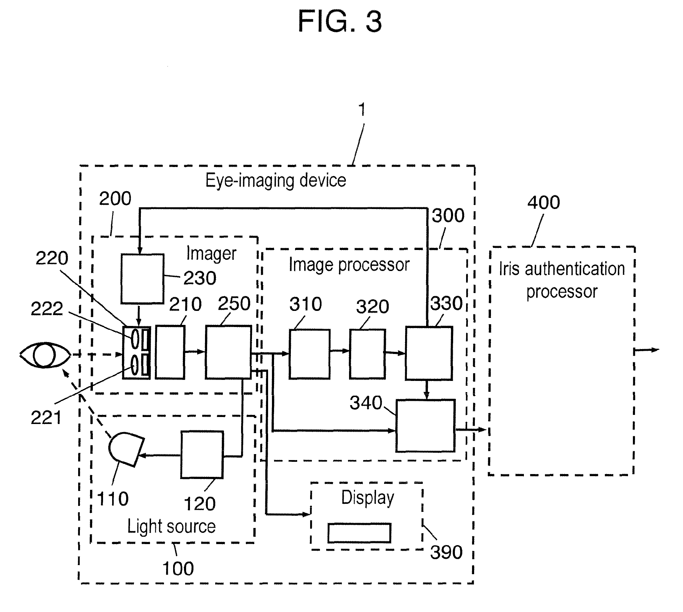

FIG. 3 shows a block diagram of eye-imaging device 1 and iris authentication processor 400 used in the first preferred embodiment of the present invention. Eye-imaging device 1 comprises: imager 200, light source 100, image processor 300, display 390. Cell-phone 10 in the first preferred embodiment comprises iris authentication processor 400 in itself to carry out iris authentication processing on eye-image data taken in imager 200 and received from image processor 300.

Light source 100 comprises LED 110 emitting near infrared light and light control 120 for LED 110 to emit a sufficient amount of light on the eye to take eye-images.

Imager 200 comprises aforementioned optical system 220, imaging element 210, preprocessor 250, and lens selector 230 to switch over lenses and filters automatically. Reflected light from a face or an eye of a subject person is input into imaging element 210 through one of wide-angle lens 222 and telephoto lens 221 of optical system 220. The input incoming light is input into preprocessor 250 as an electric signal through the process of photoelectric conversion in imaging element 210. Preprocessor 250 extracts image signal components out of the electric signal input from imaging element 210 and the data are processed such as gain controlling or the like that are required for image signals before output to image processor 300 as well as to display 390. Lens selector 230 switches over to wide-angle lens 222 or telephoto lens 221 in lenses of optical system 220 according to signals from guide checker 330 explained later.

Image processor 300 comprises: pupil detector 310, inter-pupil pixel count checker 320, guide checker 330, and authentication image storage 340. Pupil detector 310 detects positions of two pupils in a facial image taken by wide-angle lens 222 and output from preprocessor 250. To detect pupil positions in the facial image there are some conventional ways such as template matching method, contour integration method (see Japanese Patent Unexamined Publication No.H8-504979) or the like. Inter-pupil pixel count checker 320 measures the distance between two pupils in the facial image to determine the distance required for the camera to take images by comparing the distance with the distance between two pupils in the image that has been registered previously. Guide checker 330 checks whether the pupil locates in the center of the display screen and has stayed there stably for a certain period of time (for instance of the order of 0.5 seconds or so) before sending a signal to lens selector 230 to say that the eye has been finished guided. Authentication image storage 340 stores eye-images, taken by telephoto lens 221, output from preprocessor 250 and sends them to iris authentication processor 400 as the eye-images to be used for authentication.

Iris authentication processor 400 extracts iris data in coded form out of eye-image for authentication output from image processor 300 to compare the data with the data registered previously for the authenticity of the subject person.

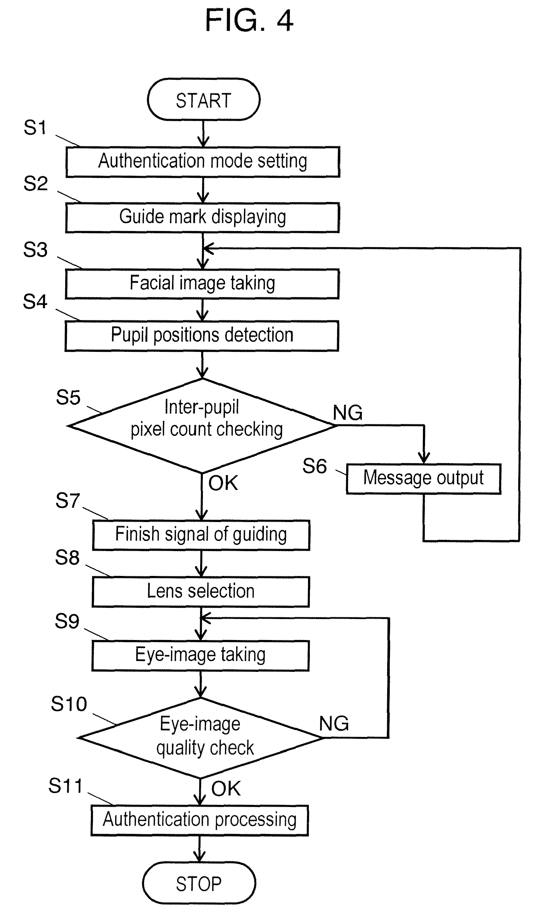

Next, the operation procedure of eye-imaging device used in the first preferred embodiment is described. Here, the user is considered to be a subject person. The flowchart in FIG. 4 shows the operation procedure of the eye-imaging device. Here, it is described that optical system 220 shall be set with wide-angle lens 222 in the initial state.

To perform the iris authentication, firstly the user shall set the operation modes of a cell-phone for authentication mode (S1). Display 390 displays a mark for instance a cross mark to guide the eye, leaving wide-angle lens 222 remained (S2). Imager 200 takes facial images of a subject person (S3). Pupil detector 310 detects positions of the two pupils in the facial image of the subject person (S4). Inter-pupil pixel count checker 320 compares the number of pixels between the two pupils with the number of pixels between two pupils of the subject person registered previously (S5). Consequently, if the number of pixels between the two pupils is too small, display 390 outputs a message saying to near the camera to the eye because the imaging distance is too large, and contrarily if the number of pixels is too large the message says the camera should be away more because the imaging distance is too small. Additionally, if the subject person's eye is not viewed at a predetermined position, or the pupil of the eye is not viewed at the position indicated by the cross mark in display 390 to guide the eye, the message says the eye should be positioned to the cross mark. A voice message using a speaker can be available instead of the displayed message. If the number of pixels between two pupils is too large or too small or no pupil is viewed at the position of the cross mark for the eye guiding, facial image must be taken again (S6).

When the number of pixels between two pupils is equal to the number of pixels between the two pupils that has been registered previously, and the pupil image has located in the cross mark position and has stayed there stably for a certain period of time (for instance of the order of 0.5 seconds), guide checker 330 outputs a signal to lens selector 230 that the guiding has finished (S7).

Upon receiving the signal that the guiding has finished, lens selector 230 switches over the lens of optical system220 from wide-angle lens 222 to telephoto lens 221 (S8). Imager 200 takes an eye-image of the subject person (S9). Checking the quality of the eye-image received, authentication image storage 340 outputs the eye-image to iris authentication processor 400 if the image quality is acceptable to be used for iris authentication. If not so, the eye-image must be taken again (S10). Iris authentication processor 400 extracts iris data in coded form out of eye-image for authentication to compare the data with the data registered previously for the authenticity of the subject person (S11).

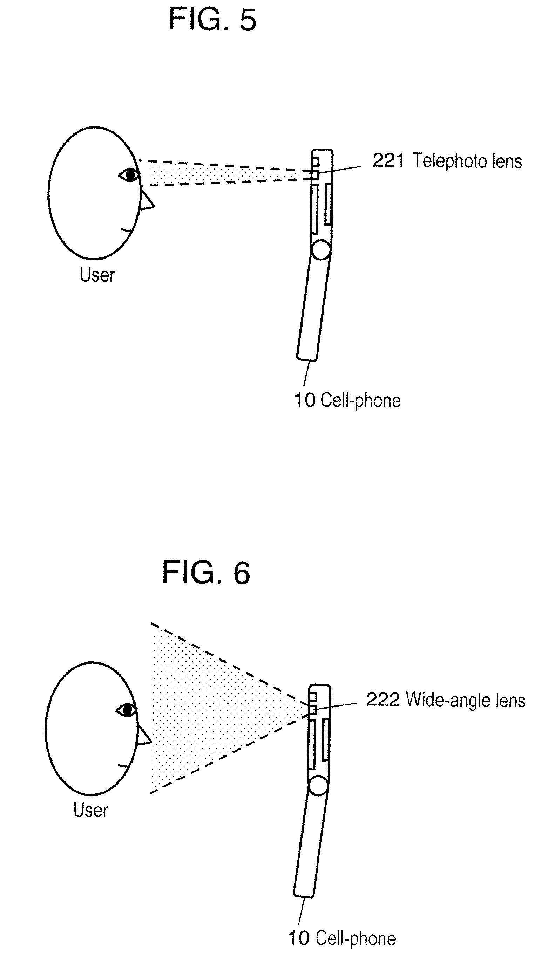

FIG. 5 shows an angle-of-view of telephoto lens 221 of the imager in the eye-imaging device used in the first preferred embodiment of the present invention, and FIG. 6 shows an angle-of-view of wide-angle lens 222 of the same. Telephoto lens 221 is to take iris images for the iris authentication, being set up the focal length of the order of 30 cm or so. The lens can take a close-up eye-image of the user himself/herself in full screen. On the other hand, wide-angle lens 222 is to take facial images for guiding the eye, but can be used as an ordinary camera for photography when used other than authentication purposes. Additionally, cell-phone 10 can be used as a digital camera or a video telephone because wide-angle lens 222 can take images of wide angle-of-view as shown in FIG. 6.

As mentioned above, the eye-imaging device of the first preferred embodiment can guide the eye easily because the facial image of a subject person taken by the wide-angle lens is displayed in the display screen, and can switch over between the wide-angle lens and telephoto lens automatically, resulting in no concern of image blurring. Additionally, the distance between the eye and camera to carry out iris authentication can be shortened to the order of 30 cm or so, a typical distance seen usually as an average distance when the cell-phone is used, the configuration can eliminate the feeling of wrongness or uneasiness in handling the camera to take eye-images and can prevent others from sensing his/her behaviors for the iris authentication. Additionally, the optical system has the configuration to switch over the two lenses only so that the production cost can be reduced significantly.

### (The second preferred embodiment)

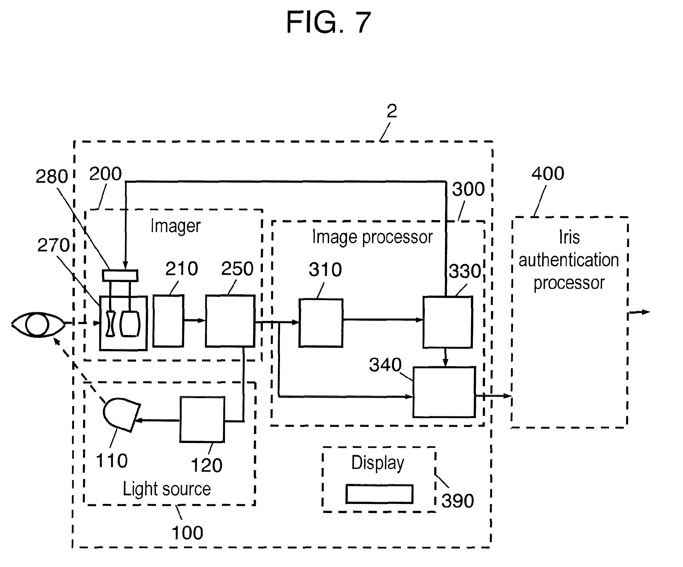

FIG. 7 shows a block diagram of eye-imaging device 2 and iris authentication processor 400 used in the second preferred embodiment of the present invention. The components used in eye-imaging device 2 of the second preferred embodiment that are similar to the components in the first preferred embodiment have the same reference marks as shown in FIG. 3. Eye-imaging device 2 comprises: imager 200, light source 100, image processor 300, and display 390. Additionally, like the configuration in the first preferred embodiment, cell-phone 10 comprises iris authentication processor 400 in itself to carry out iris authentication processing on eye-image data taken in imager 200 and received from image processor 300.

Eye-imaging device 2 used in the second preferred embodiment differs significantly from the first preferred embodiment in the configuration that optical system 270 provided in front of imager 210 is composed of a zoom lens. Imager 200 comprises: optical system 270 composed of the zoom lens, imager 210, preprocessor 250, and zoom lens driver 280. Zoom lens driver 280 has a function to switch over optical system 270 of imager 200 to a wide-angle mode or a telephoto mode automatically by driving the zoom lens according to signals from guide checker 330 described later and a function of auto-focusing as well. The zoom lens structure and the auto-focusing function can be realized using known technologies.

Image processor 300 comprising pupil detector 300, guide checker 330, and authentication image storage 340, does not need inter-pupil pixel count checker used in the first preferred embodiment any more because zoom lens driver 280 has a function of auto-focusing as well.

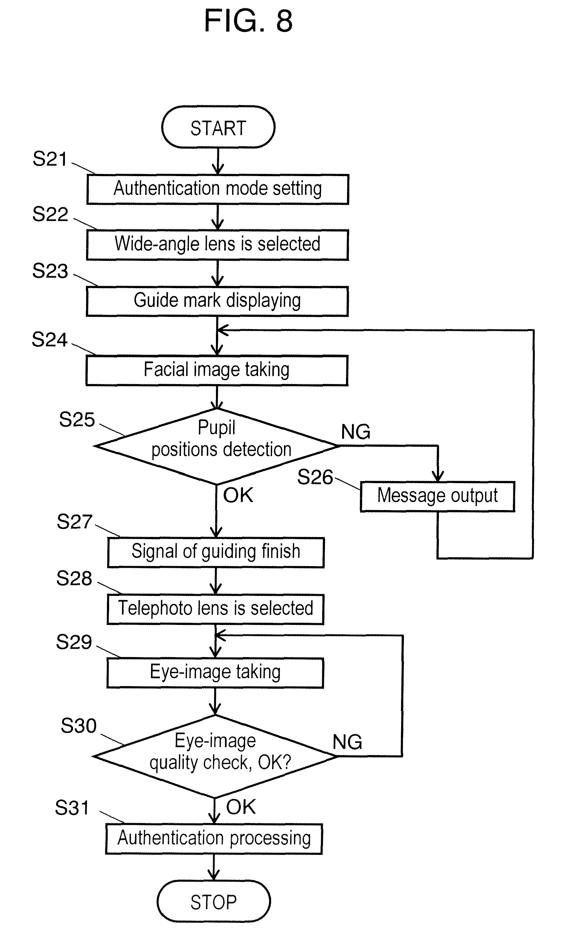

Next, the operation procedure of eye-imaging device used in the second preferred embodiment is described. The flowchart in FIG. 8 shows the operation procedure of the eye-imaging device.

To perform the iris authentication, firstly the user shall set up operation modes of the cell-phone for an authentication mode (S21). Then, zoom lens driver 280 sets up the zoom lens for wide-angle (S22). Display 390 shows a cross mark to guide the eye (23). Imager 200 takes facial images of the subject person (S24). Pupil detector 310 detects positions of the two pupils in the facial image of the subject person (S25). At this time, if the pupil of the subject person's eye is not viewed at the position indicated by a cross mark in display 390 for the eye guiding, a message is output saying the eye should be positioned to the cross mark. A voice message using a speaker can be available instead of the output message. If no pupil is viewed at the position of the cross mark for eye guiding, facial image must be taken again (S26). When the pupil image of the face has stayed stably without blurring in the cross mark position for a certain period of time (for instance of the order of 0.5 seconds), guide checker 330 outputs a signal to zoom lens driver 280 that the guiding has finished (S27). Upon receiving the signal that the guiding has finished, zoom lens driver 280 sets up the zoom lens of optical system 270 for telephoto lens and focuses the camera (S28). Imager 200 takes an eye-image of the subject person (S29). Checking the quality of the eye-image received, authentication image storage 340 outputs the eye-image to iris authentication processor 400 if the image quality is acceptable for iris authentication. If not so, the eye-image must be taken again (S30). Iris authentication processor 400 extracts iris data in coded form out of eye-image for authentication to compare the data with the data registered previously for the authenticity of the subject person (31).

As mentioned above, the eye-imaging device of the second preferred embodiment can set the distance between the eye and camera for iris authentication to the order of 30 cm or so, enabling to eliminate the feeling of wrongness or uneasiness in handling the camera and to prevent others from sensing his/her behaviors for the iris authentication. Additionally, the time to taking images can be shortened according to the user's proficiency by narrowing the angle-of-view to guide the eye, because the zoom lens can set the angle-of-view freely. Moreover, the eye position can be adjusted easily as soon as it begins to deflect because the user can always check the eye position by watching zooming images continuously on display 390. In the case of imager having a zoom lens as described in the second preferred embodiment, the user can set the distance between his/her eye and the camera for iris authentication in a certain ranges according to the user's request.

### (The third preferred embodiment)

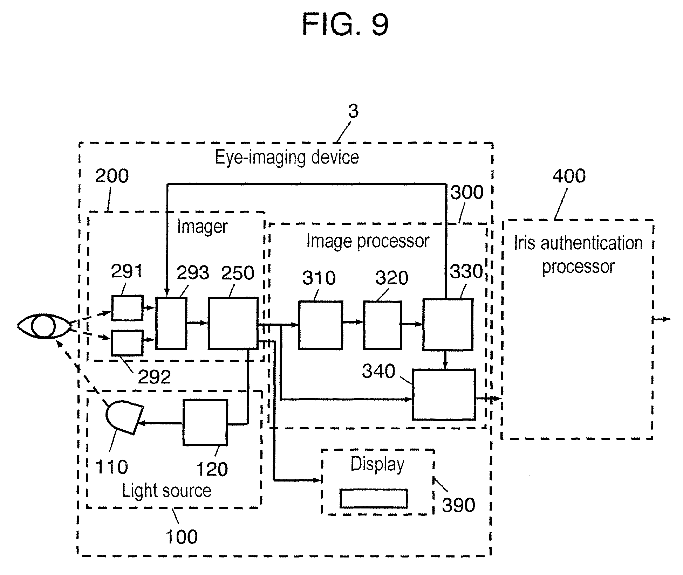

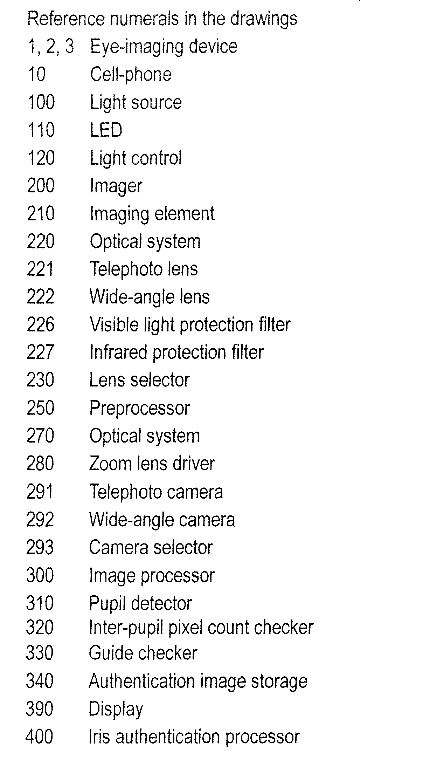

FIG.9 shows a block diagram of eye-imaging device 3 and iris authentication processor 400 used in the third preferred embodiment of the present invention. The components used in eye-imaging device 3 of the third preferred embodiment that are similar to the components used in the first preferred embodiment have the same reference marks as shown in FIG. 3. Eye-imaging device 3 comprises: imager 200, light source 100, image processor 300, and display 390. Additionally, like in the first preferred embodiment, cell-phone 10 comprises iris authentication processor 400 in itself to carry out iris authentication processing on eye-images received from image processor 300.

Eye-imaging device 3 used in the third preferred embodiment differs significantly from the first preferred embodiment in the configuration that imager 200 comprises: telephoto camera 291, wide-angle camera 292 and camera selector 293 to select these cameras. Camera selector 293 switches over between telephoto mode and wide-angle mode of imager 200 by switching over between telephoto camera and wide-angle camera electrically. Camera selector 293 switches over between telephoto camera 291 and wide-angle camera 292 of imager 200 according to signals from guide checker 330, like the case of lens selector 230 in the first preferred embodiment.

The operation procedure of eye-imaging device of the third preferred embodiment is similar to that of the first preferred embodiment, and the description is omitted.

As mentioned above, imager 200 of the third preferred embodiment switches over the cameras automatically and electrically without using any mechanical movable parts, resulting in the performance with high reliability and less power consumption.

When using the portable terminal device with the eye-imaging device of the present invention, a certain distance between the eye and camera can be remained to perform the iris authentication, which can eliminate the feeling of wrongness or uneasiness in handling the camera and can prevent others from sensing his/her behaviors for the iris authentication.

### INDUSTRIAL APPLICABILITY

The eye-imaging device of the present invention can take eye-images keeping a certain distance between the eye and camera, which can eliminate the feeling of wrongness or uneasiness in handling the camera and can prevent others from sensing his/her behaviors for the iris authentication. The eye-imaging device, therefore, is useful for use in eye-imaging devices for iris authentication installed into downsized equipment such as portable terminal device, and in particular for use in portable terminal device capable of taking both wide-angle images such as facial images or the like and narrow-angle images such as iris images or the like.

## Claims

1. An eye-imaging device comprising:
an imager capable of switching over between a telephoto mode to take a subject person's eye-images and a wide-angle mode to take the subject person's facial images, keeping a certain distance to the subject person's eye;
a display to display images taken by the wide-angle mode;
a guide checker to check automatically that the subject person's eye has been guided to a predetermined position according to the image taken by the wide-angle mode; and
a selector to switch over the imager automatically from the wide-angle mode to the telephoto mode when the guide checker checks that the subject person's eye has been guided to the predetermined position.

2. The eye-imaging device of claim 1, wherein
the imager has a telephoto lens and a wide-angle lens, and
the selector is a lens selector to switch over between the telephoto mode and the wide-angle mode by switching over between the telephoto lens and the wide-angle lens.

3. The eye-imaging device of claim 1, wherein
the imager has a zoom lens, and
the selector is a zoom lens driver to switch over between the telephoto mode and the wide-angle mode by driving the zoom lens.

4. The eye-imaging device of claim 1, wherein
the imager has a telephoto camera and a wide-angle camera, and
the selector is a camera selector to switch over between the telephoto mode and the wide-angle mode by switching over between the telephoto camera and the wide-angle camera.

5. A portable terminal device having the eye-imaging device of one of claim 1 to 4.
